# EUROPEAN PATENT APPLICATION

(11) **EP 4 548 928 A1**
(43) Date of publication of application: **07.05.2025**
(21) Application number: 23383111.4
(22) Date of filing: 31.10.2023
(51) Int. Cl.: A61K 39/05, A61K 39/12, A61P 31/20

(54) **ROTHIA NASIMURIUM OR IMMUNOMODULATORY FRACTION THEREOF IN THE PREVENTION AND/OR TREATMENT OF AN INFECTION OR A NON-INFECTIOUS DISEASE IN A SUBJECT**

(71) Applicant: Institut de Recerca i Tecnologia Agroalimentaries (IRTA), 08140 Caldes de Montbui (Barcelona) (ES); Universitat Autònoma de Barcelona, 08193 Cerdanyola del Valles (ES)
(72) Inventor: RODRÍGUEZ GONZÁLEZ, Fernando, 08140 CALDES DE MONTBUI (Barcelona) (ES); ARAGÓN FERNÁNDEZ, Virginia, 08140 CALDES DE MONTBUI (Barcelona) (ES); ARGILAGUET MARQUÉS, Jordi, 08140 CALDES DE MONTBUI (Barcelona) (ES); CORREA FIZ, Florencia, 08140 CALDES DE MONTBUI (Barcelona) (ES); ZHANG, JINYA, 08193 CALDES DE MONTBUI (Barcelona) (ES); MARTÍNEZ MARTÍNEZ, Jorge, 08193 Bellaterra (Cerdanyola del Valles) Barcelona (ES)
(74) Representative: Ungria López, Javier

(57) **Abstract**

The invention relates to a strain of *Rothia nasimurium,* live or inactivated, or an immunomodulatory fraction thereof, wherein the strain of *Rothia nasimurium* has "Colección Española de Cultivos Tipo" (CECT) accession number 30828. The invention also relates to a live or inactivated strain of *Rothia nasimurium* or an immunomodulatory fraction thereof, for use in the prevention and/or treatment of an infection or a non-infectious disease in a subject. A pharmaceutical product, a veterinary product, a feed product, and a nutritional product are also disclosed, wherein the product comprises a live or inactivated strain of *Rothia nasimurium.* Additionally, the invention refers to a use of *Rothia nasimurium* as an adjuvant. Finally, the invention teaches a composition comprising: (a) a live attenuated African swine fever virus (ASFV); and (b) a live or inactivated strain of *Rothia nasimurium,* or immunomodulatory fraction thereof, and the use of said composition in the prevention and/or treatment of African Swine Fever (ASF).

## Description

### FIELD OF THE INVENTION

The invention pertains to the field of immunology and provides protection strategies against diseases. In particular, the invention discloses methods for the treatment and/or prevention of an infectious or non-infectious disease in a subject, and more specifically treatment and/or prevention of African Swine Fever (ASF) in domestic pigs.

### BACKGROUND ART

African swine fever (ASF) is a disease of obligatory declaration to the World Organization for Animal Health (WOAH) that exclusively affects domestic pigs and wild boars (*Sus scrofa* species) and has become the number one problem for the swine industry, threatening global commercial trading (www.woah.int). Described for the first time in Kenya 1921 as a lethal disease that affected the first domestic pigs brought to the region, ASF spread rapidly within the African continent, and still today is considered an endemic problem that contributes to the underdevelopment of the swine industry of the region and to poverty. African swine fever virus (ASFV), the causative agent of ASF, is a large and complex double stranded DNA virus (>170kpb) that encodes more than 150 proteins (Alejo et al., 2021). ASFV was exported for the first time out of Africa to Portugal in the decade of 1950 and, from there, the virus spread to Spain and sporadically to other European and American countries. The situation in the Iberian Peninsula was extremely dramatic becoming endemic for more than 40 years and provoking the prohibition of exportation of pork-derived products until 1997, when the virus was eradicated from Continental Europe. However, only 10 years later (in the year 2007), the virus reemerged in Europe, this time in Georgia and since then, it has expanded West and East to the point that all continents are nowadays affected.

Lack of commercial vaccines hinders effective control of ASF and today many pork producers have been banned from exporting any pork products, including China, Germany, and Italy. So far, only live attenuated vaccines (LAVs) have demonstrated high protective efficacy against experimental, lethal ASFV challenge, albeit safety problems have hampered their implementation in the field. The inventors, who investigate ASF vaccine development, have achieved a series of advances in this field. They have recently formalized the transfer of vaccine prototype BA71ΔCD2 (based on a recombinant live virus lacking the CD2v antigen). The inventors' recent confirmation that intranasal inoculation of BA71ΔCD2 significantly improves safety compared to intramuscular administration of the vaccine, has allowed advancing the prosecution of the vaccine's official registration. The cross-protective immunity induced in pigs upon intranasal vaccination with BA71ΔCD2 is associated with the activation of both lymphocytes and myeloid cells. More specifically, *in vitro* ASFV-specific stimulation of cells from immunized pigs revealed a robust Th1 response, defined by the presence of poly-functional CD4+CD8+ T cells as well as the expansion of cytotoxic T cells through antigen presentation by cross-presenting dendritic cells (cDC1). Interestingly, this adaptive immune response induced an IFNy-dependent positive feedback regulation of innate immunity, characterized by rapid activation of inflammation.

In any case, there is still an urgent need in the art to provide effective preventive measures and treatment options that could be used to control ASF. With this aim, the present invention discloses the use of a strain of the genus *Rothia* for immunomodulatory purpose in the control of ASF, specifically *Rothia nasimurium* that has been never disclosed in the prior art.

Although the genus *Rothia* has been used in the biotechnological field, no disclosure teaches or suggests this specific strain or the intended use. For instance, document US2018333440A1 discloses a method of treatment for gut dysbiosis, asthma, allergy, atypical dermatitis, or for altering or colonising the gastrointestinal tract in humans; said method including administering a bacterial composition comprising *Faecalibacterium* and *Lachnospira,* among others bacteria, including the genus *Rothia*). No medical use in animals of the genus *Rothia* is disclosed. Document AU2021204574A1 refers to a method for reducing inflammation in a subject comprising a composition with a bacterial population, which includes one or more species of the genus *Blautia*, and which may also contain *Rothia,* i.e. a method, with a composition comprising more than one species, including *Rothia.* In document KR20210133986A a pharmaceutical composition of an isolate of a bacterial membrane preparation, and/or a bacterium is disclosed, wherein the membrane and/or bacterium could be *Rothia.* However, no mention is made to *Rothia nasimurium,* and the present invention discloses an immunostimulatory fraction of *Rothia nasimurium* which is obtained from an aqueous solution after washing a whole live cell culture of the said bacteria, which should not contain bacterial membrane fragments.

### SUMMARY OF THE INVENTION

The inventors have found that a strain of *Rothia nasimurium* isolated from the microbiota of warthogs (*Phacochoerus africanus*) possesses immunomodulatory properties, such that domestic pigs treated with said strain of *Rothia nasimurium* may acquire protection against infection, particularly against infection with the African swine fever virus (ASFV). The inventors have also determined that a specific fraction of the strain of *Rothia nasimurium* maintains similar immunomodulatory properties. Finally, the inventors have also confirmed that the strain of *Rothia nasimurium* (or the immunomodulatory fraction thereof) may be used as a vaccine adjuvant, particularly when used in combination with a live attenuated vaccine (LAV) against African swine fever (ASF).

Thus, in a first aspect the invention relates to a strain of *Rothia nasimurium* or an fraction thereof, wherein the strain of *Rothia nasimurium* has "Colección Española de Cultivos Tipo" (CECT) accession number 30828, and wherein both, not only the strain but also the fraction thereof, presents immunomodulatory properties.

The strain of *Rothia nasimurium* may be a live strain or an inactivated strain, not only for this object of invention but also for the following objects that will be disclosed.

In a second aspect, the invention relates to a (live or inactivated) strain of *Rothia nasimurium* or an immunomodulatory fraction thereof, for use in the prevention and/or treatment of a disease in a subject, said disease being any potential infection or any non-infectious disease.

In a third aspect, the invention relates to a product that comprises a (live or inactivated) strain of *Rothia nasimurium,* or immunomodulatory fraction thereof, said product being selected from the group consisting of: a pharmaceutical product, a veterinary product, a feed product, and a nutritional product, such as a prebiotic, probiotic or postbiotic product. It is understood in the scope of the present disclosure that a prebiotic is a substrate selectively utilized by host microorganisms conferring a health benefit, whilst the probiotics are live microorganisms that, when administered in adequate amounts, confer a health benefit on the host. A postbiotic is any factor resulting from the metabolic activity of a probiotic or any released molecule capable of conferring beneficial effects to the host in a direct or indirect way.

In a fourth aspect, the invention also relates to the use of a strain of *Rothia nasimurium,* or immunomodulatory fraction thereof, as an adjuvant. The strain can be a live strain or an inactivated strain. In a preferred case, the strain is used as a vaccine adjuvant.

In a fifth aspect, the invention relates to a composition comprising: (a) a live attenuated African swine fever virus (ASFV); and (b) a (live or inactivated) strain of *Rothia nasimurium,* or immunomodulatory fraction thereof.

In a final aspect, the invention relates to the use of a composition comprising: (a) a live attenuated African swine fever virus (ASFV); and (b) a (live or inactivated) strain of *Rothia nasimurium,* or immunomodulatory fraction thereof for the prevention and/or treatment of African Swine Fever (ASF). In this sense, the invention is related to
- a method for preventing and/or treating African Swine Fever (ASF), as well as
- the use of the composition that comprises (a) a live attenuated African swine fever virus (ASFV); and (b) a (live or inactivated) strain of *Rothia nasimurium,* or immunomodulatory fraction thereof for the manufacture of a medicament for the prevention and/or treatment of African Swine Fever (ASF).

### BRIEF DESCRIPTION OF THE FIGURES

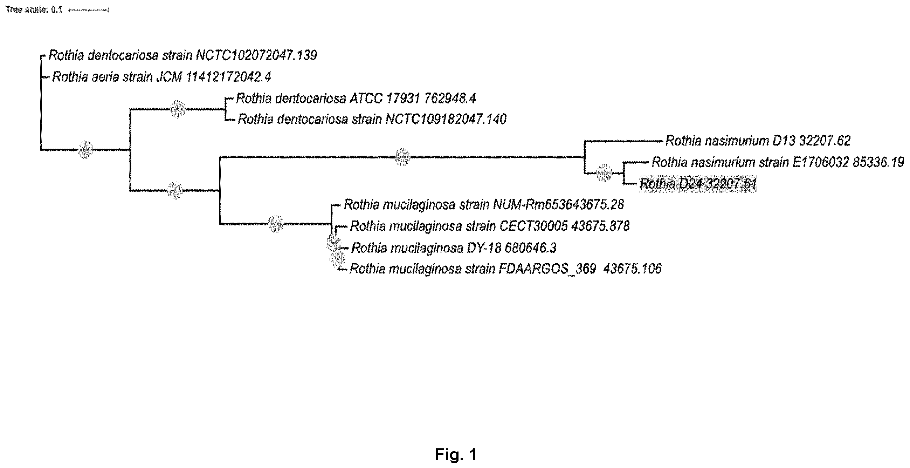

Figure 1: Phylogenetic tree of warthog *Rothia nasimurium* D24 genome with available full genomes within *Rothia* genus, with 1,000 bootstrap created with RAxML and edited with iTOL.

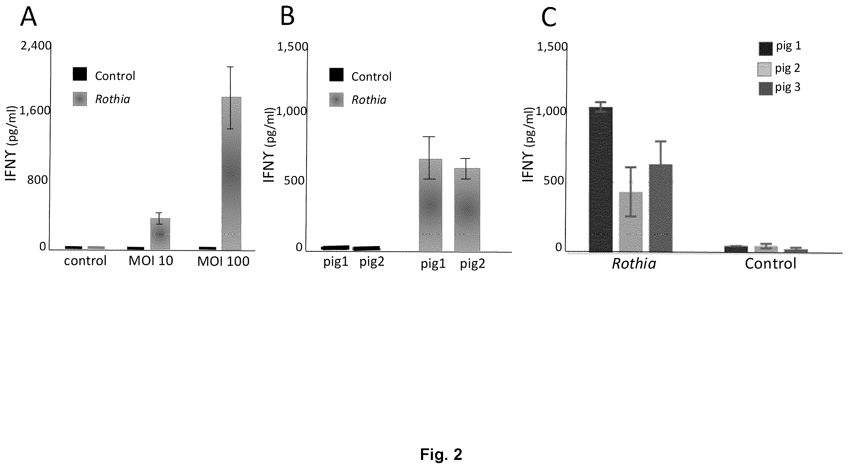

Figure 2: Warthog *Rothia nasimurium* D24 promotes the induction of IFNγ in vitro. A) *Rothia nasimurium* D24 stimulates newborn pig trachea (NPTr) cells to secret IFNγ in a dose dependant manner (MOI: multiplicity of infection); B) *Rothia nasimurium* D24 stimulates peripheral blood mononuclear cells (PBMCs) isolated from two different pigs to secret IFNγ; C) *Rothia nasimurium* D24 stimulates Peyer's patches (PP) & mesenteric lymph nodes (MLN) isolated from three different pigs. IFNγ was quantified by ELISA from cell supernatants after 24-hour incubation. Average and standard deviation values from three independent assays are plotted.

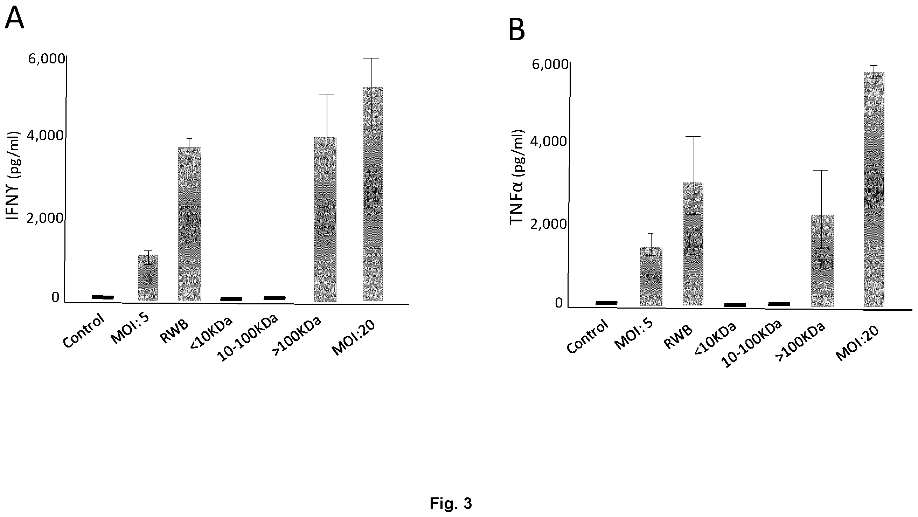

Figure 3: Porcine alveolar macrophages (PAMs) secrete IFNγ (A) and TNFα (B) in response to warthog *Rothia nasimurium* D24 *in vitro* stimulation. PAMs were overnight incubated with either: alive warthog *Rothia nasimurium* D24 at MOI:5 or 20, *"Rothia's* wash" (RW) or the different fractions from RW, and the cell culture supernatants were collected after 24 hours and used to measure the presence of IFNγ (A) and TNFα (B) by ELISA. Average and standard deviation values obtained after stimulation of PAMs from three different animals are plotted.

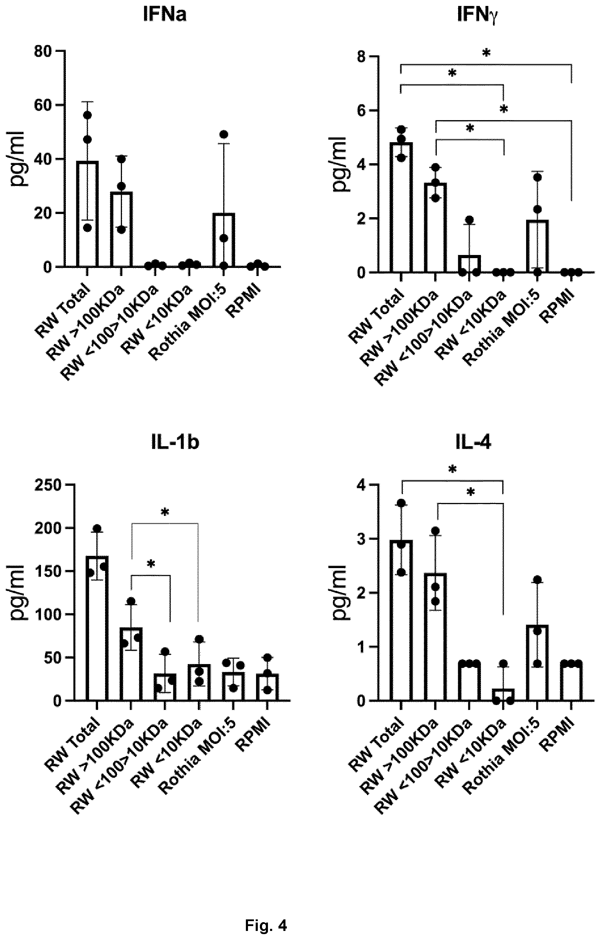

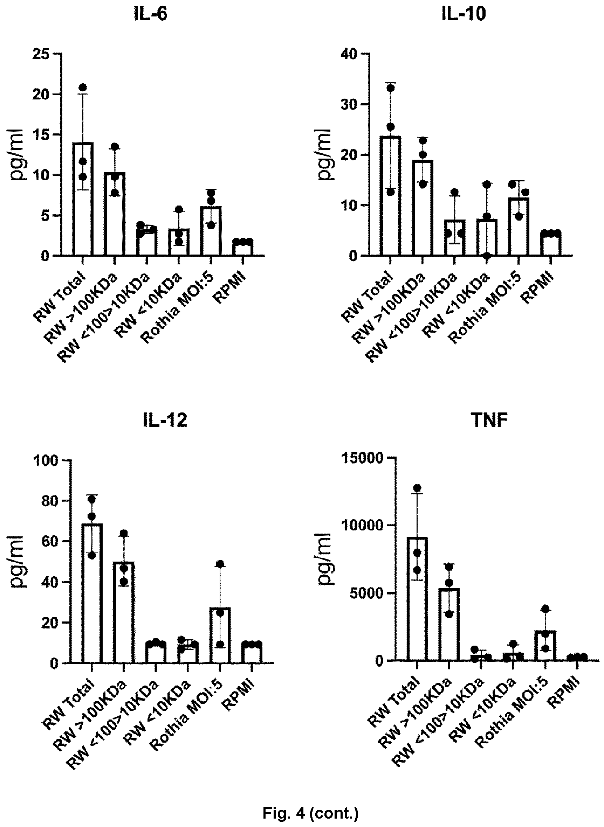

Figure 4: PAMs secrete pro- and anti-inflammatory cytokines in response to warthog *Rothia nasimurium* D24 *in vitro* stimulation. PAMs were overnight incubated with either: alive warthog *Rothia nasimurium* D24 at MOI:5, RW or the different fractions from RW and the supernatants used to measure the presence of: IFNα, TNFα, IL6, IFNγ, IL12, IL1β, IL4 and IL10 by LUMINEX. Average and standard deviation values obtained after stimulation of PAMs from three different animals are plotted. The values of IFNγ are underestimated in the LUMINEX (note from the providers). The "*" in the figure means statistically significant.

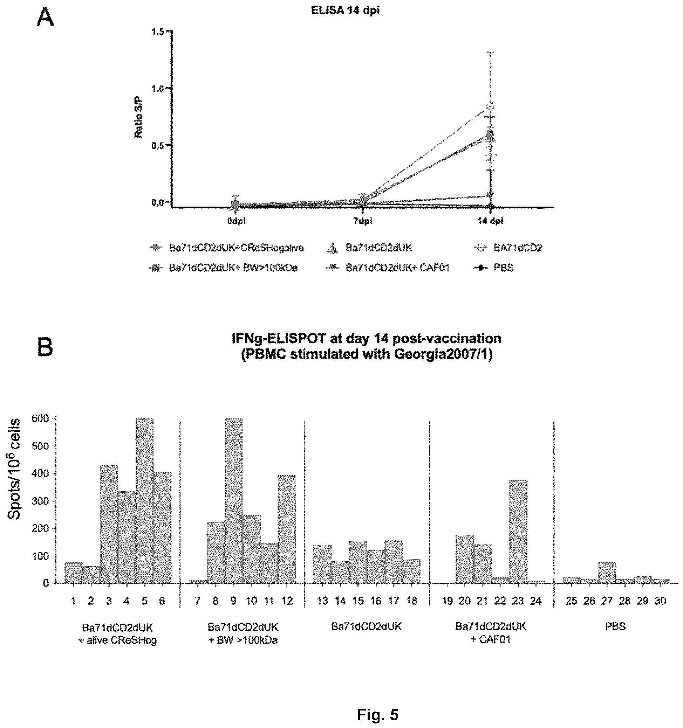

Figure 5: The Th1-like immune responses induced after intranasal inoculation of BA71ΔCD2ΔUK is improved by using warthog *Rothia nasimurium* D24 as adjuvant.
   5A) ASFV specific IgGs detected by ELISA at different times after intranasal inoculation of BA71ΔCD2ΔUK alone or in the presence of CAF01 adjuvant. Average and standard deviations for each group are shown.
   5B) ASFV specific T-cells detected in PBMCs by IFNγ-ELISPOT at 14 days post inoculation. Each column in Figure 5B corresponds to individual pigs.

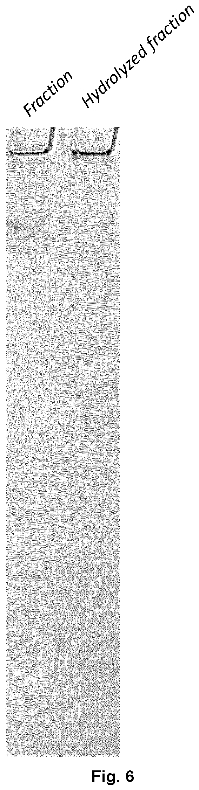

Figure 6: Sodium dodecyl sulphate polyacrylamide gel electrophoresis (SDS-PAGE) of the fraction with immunomodulatory properties (IM fraction) of the *Rothia nasimurium* D24. The fraction was run in the gel before (left) and after (right) hydrolysis with acetic acid, and stained with periodic acid-Schiff (PAS) reagent.

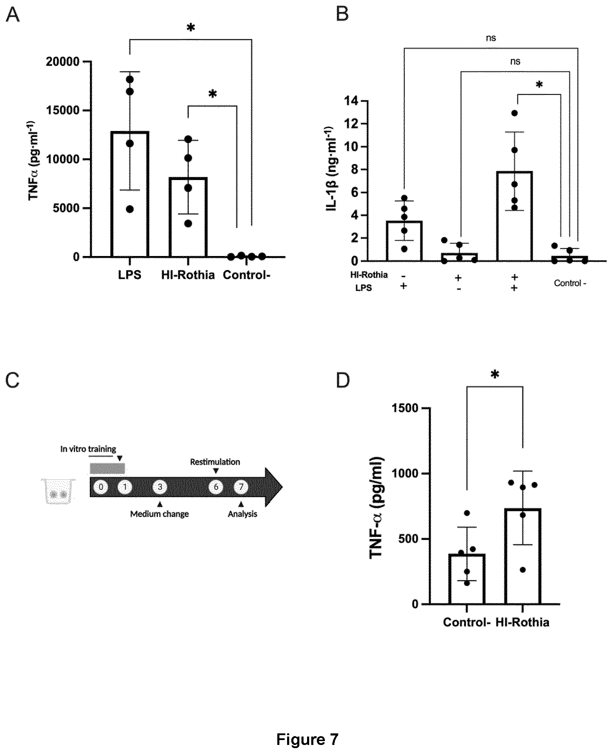

Figure 7: (A) Levels of TNFα measured by ELISA from PAMs stimulated for 24 hours with heat inactivated Rothia (HI-Rothia; MOI 50) or with LPS (10 mg/ml). (B) Levels of IL1b measured by ELISA in supernatants from PAMs stimulated for 24 hours with inactivated Rothia (HI-Rothia; MOI 50) alone or in combination with LPS (50 ng/ml), to test its capacity to activate inflammasome. (C) Experimental plan to test induction of Trained Immunity in porcine alveolar macrophages in vitro. (D) Levels of TNFα measured by ELISA in supernatants from PAMs trained with inactivated Rothia (HI-Rothia; MOI 5) or Frac X (or not trained as negative control) and re-stimulated at day 6 with LPS (10 ng/mL). The "*" in the figure means statistically significant.

### DETAILED DESCRIPTION OF THE INVENTION

As explained above, the inventors have found that a strain of *Rothia nasimurium,* either live or inactivated, isolated from the microbiota of warthogs (*Phacochoerus africanus*) possesses immunomodulatory properties, such that domestic pigs treated with said live or inactivated strain of *Rothia nasimurium* may acquire protection against infection, particularly against infection with the African swine fever virus (ASFV). The inventors have also determined that a specific fraction of the strain of *Rothia nasimurium* maintains similar immunomodulatory properties. Finally, the inventors have also confirmed that the strain of *Rothia nasimurium* (or the immunomodulatory fraction thereof) may be used as a vaccine adjuvant, particularly when used in combination with a live attenuated vaccine (LAV) against African swine fever (ASF).

Thus, in a first aspect the invention relates to a strain of *Rothia nasimurium* or an immunomodulatory fraction thereof, wherein the strain of *Rothia nasimurium* has "Colección Española de Cultivos Tipo" (CECT) accession number 30828. The CECT is an International Depositary Authority under Article 7 of The Budapest Treaty. The address of the CECT is Edificio 3 CUE, Parc Cientific Universitat de València, Catedrático Augustin Escardino, 9, 46980 Paterna (Valencia). The strain of *Rothia nasimurium* was isolated from faecal material from warthogs kept at the Barcelona ZOO. The applicant of the present invention is also the depositary of the strain at the International Depositary Authority.

In the context of the instant invention, the term *"Rothia nasimurium"* refers to a species of the genus *Rothia,* wherein the genus *Rothia* is identified in the NCBI database (see Figure 1) by Taxonomy ID: 32207 (NCBI:txid32207), and wherein the species *Rothia nasimurium* is identified in the NCBI database by Taxonomy ID: 85336 (NCBI:txid85336). The term "strain" as used herein refers to a genetic variant, a subtype or a specific culture within a biological species. Thus, the strain of *Rothia nasimurium* in the context of the invention is a certain biological form of the bacteria that can be distinguished from other strains of *Rothia nasimurium* by means of differences in the genome, differing isoforms of surface proteins, different antimicrobial resistance behaviour, etc.

Also in the context of the instant invention, the term "fraction" refers to a part of the *Rothia nasimurium* that retains its immunomodulatory capabilities, i.e. it is an immunomodulatory fraction. This fraction is what the inventors have called *"Rothia's* wash" (RW), obtained after collecting in PBS the *Rothia* colonies grown on agar plates, then centrifuged to pellet the bacteria and finally filtered through a 0.2 µ filter (RW in Figure 3A). A simple fractionation of the RW using Amicon Ultra-15 Centrifugal Filter Unit columns (Millipore #UFC9100), allowed the inventors to confirm that the RW fraction of higher molecular weight (>100KDa in Figure 3A) maintains the immunomodulatory (IM) capabilities, while RW fractions with molecular weights between 10-100KDa and <10KDa were not able to stimulate PAMs to secrete IFNY (Figure 3A). Exactly these same results were observed when measuring tumour necrosis factor alfa (TNFα) in the same PAMs supernatants (Figure 3B), confirming the IM nature of warthog *Rothia* and the high molecular weight fraction (>100KDa) of RW (from now on named IM fraction). This IM fraction does not contain detectable protein and it is composed by carbohydrates (sugars) and lipids. When run in an SDS-gel and stained with PAS reagent (specifically staining polysaccharides) the content of the fraction it is revealed as a specific band of high molecular weight (see left line in attached figure 6). The fact that the polysaccharide does not enter the gel when hydrolysed with 2% acetic at 100°C seems to confirm that the immunomodulatory compound is of glycolipid nature.

In a preferred embodiment, the invention refers to cells of *Rothia nasimurium,* preferably whole cells. In a particular embodiment, the cells can be whole alive cells. In another particular embodiment, the composition comprises killed cells (i.e. inactivated bacteria). The bacteria may be killed by any known means, such as heat or proteinase K treatment, among other treatments. The composition may comprise cell lysates or extracts of *Rothia nasimurium,* the lysis or extraction of the cells not affecting the immunomodulatory compounds (identified of glycolipid nature). It needs to be specified that a fraction cell is different from a cell lysate. Cell lysate are dead bacteria, whilst the fraction is a part of the bacterial wash after phase-separation by molecular weight.

The cells may also be lysed by means of chemical lysis or mechanical lysis. In mechanical lysis, cell membrane is physically broken down by using shear force. This method combines high throughput and relatively higher lysing efficiency. As a person skilled in the present art would readily understand, the term mechanical lysis includes techniques such as lysis by High Pressure Homogenizer (HPH), sonication and Bead Mill lysis, among others known techniques.

In a second aspect, the invention relates to a (live or inactivated) strain of *Rothia nasimurium* or an immunomodulatory fraction thereof, for use in the prevention and/or treatment of a disease in a subject, said disease being any infection or any non-infectious disease. Similarly, the invention relates to the use of a live or inactivated strain of *Rothia nasimurium* or an immunomodulatory fraction thereof, for the manufacture of a medicament for the prevention and/or treatment of a disease in a subject, and to a method for the prevention and/or treatment of a disease in a subject, wherein the method comprises administering a strain of *Rothia nasimurium* or an immunomodulatory fraction thereof to a subject in need thereof. In the context of the present invention, the general term "disease" should be understood as any infection or any non-infectious disease.

As used herein, the terms "preventing", "prevention", and the like, refer to the capacity of a given substance or composition, intended for use as a medicament, to avoid, minimise or difficult the onset or development of an infectious disease. The term "preventive treatment", as used herein, refers to any type of therapy, which is aimed at preventing or reducing susceptibility to any clinical condition as described herein. In the context of the invention, the term "treatment" refers to a prophylactic treatment of a disorder or a condition in a subject (i.e. a therapy to reduce susceptibility to a clinical condition in a subject). Thus, terms such as "treatment", "treating" and the like refer to obtaining a desired medical, pharmacologic or physiologic effect, covering any prophylactic treatment of a pathological condition in terms of completely or partially preventing a disorder or symptom thereof.

The strain of *Rothia nasimurium* of the invention, live or inactivated, or the immunomodulatory fraction thereof, is used for the prevention and/or treatment of a disease, which may be an infectious disease or a non-infectious disease. An infectious disease is caused by the infection of an agent. The term "infection", as used herein, relates to invasion by bacteria or other microorganisms, referring to an undesired presence and proliferation of pathogenic microbes in a host organism. It includes the excessive growth of microbes that are usually present in the body of the host organism. Specifically, a microbial infection can be any situation in which the presence and proliferation of a microbial population causes damage to said host organism. Thus, a microbial infection exists when excessive numbers of a microbial population are present in or on the host organism's body, or when the effects of the presence and proliferation of a microbial population is damaging the cells or other tissue of the host organism. In a particular embodiment of the invention, the infection can be an infection caused by any pathogen: including bacteria, viruses, fungi, parasites, among other pathogens.

In another particular embodiment, the invention can be used to fight a non-infectious disease treatable (or preventable) by immunomodulating the host, including but not limited to cancer, autoimmune disorder, allergic reactions...

In a particular embodiment, the infection to be treated or to be prevented with the live or inactivated strain of *Rothia nasimurium* of the invention, or immunomodulatory fraction thereof, is a bacterial infection. The terms "bacterium", "bacteria" or "bacterial infection" refer to bacterial cells capable of causing disease in a host organism, as well as producing infection-related symptoms in the infected host organism, such as fever or signs of inflammation. Bacteria cells may be selected from the group consisting of: negative, positive, and/or not-classified under Gram staining, such as Mycobacterium and Mycoplasmas. In one embodiment the bacteria are Gram-negative bacteria. In another embodiment the bacteria are Gram-positive bacteria. In another embodiment the bacteria are Gram-positive bacteria together with Gram-negative bacteria. In another embodiment there is only one single bacteria species infecting or causing disease. In yet another embodiment there are different bacteria species from one bacteria genus, infecting or causing disease. In still another embodiment there are different bacteria species from different bacteria genus, infecting or causing disease. In another particular embodiment the infection is a bacterial infection caused by a bacterium from the genus selected from the group consisting of *Acinetobacter, Actinobacillus, Aeromonas, Aggregatibacter, Agrobacterium, Anaplasma, Bacillus, Bergeyella, Bibersteinia, Bordetella, Borrelia, Brucella, Burkholderia, Campylobacter, Chlamydia, Chromobacterium, Clostridium, Corynebacterium, Cyanobacteria, Enterobacter, Enterococcus, Erwinia, Escherichia, Francisella, Fusobacterium, Glaesserella, Haemophilus, Helicobacter, Klebsiella, Lactobacillus, Legionella, Listeria, Mannhemia, Mesomycoplasma, Micrococcus, Moraxella, Mycobacteria, Mycoplasma, Neisseria, Nitrosomas, Nocardia, Obesumbacterium, Pantoea, Pasteurella, Pediococcus, Porphyromonas, Prevotella, Proteus, Pseudomonas, Ralstonia, Rickettsia, Rhisobium, Rhodobacter, Salmonella, Serratia, Shigella, Staphylococcus, Streptococcus, Tannerella, Treponema, Tsukamurella, Vibrio, Xenorhabdus, Yersinia* and combinations thereof. In particular embodiments, the bacterial infection is caused by bacteria from the genus *Salmonella, Escherichia, Clostridium* and *Streptococcus.* In particular embodiments of the invention, the infection is an infection by a pathogenic bacteria selected from the group consisting of *Salmonella typhi, Salmonella paratyphi, Salmonella typhimurium, Salmonella enteritidis, Escherichia coli, Clostridium perfringens* and *Streptococcus suis.*

In another particular embodiment, the infection to be treated or to be prevented with the strain of *Rothia nasimurium* of the invention, or immunomodulatory fraction thereof, is a virus infection. More preferably, the virus infection is caused by a virus from the genus selected from the group consisting of: Avian herpesvirus, Avian influenza, Avian leukosis virus, Avian paramyxoviruses, Border disease virus, Bovine coronavirus, Bovine ephemeral fever virus, Bovine herpes viruses, Bovine immunodeficiency virus, Bovine leukemia virus, Bovine parainfluenza virus 3, Bovine respiratory syncytial virus, Bovine viral diarrhea virus (BVDV), BVDV Type I, BVDV Type II, Canine adenovirus, Canine coronavirus (CCV), Canine distemper virus, Canine herpes viruses, Equine herpes viruses, Canine influenza virus, Canine parainfluenza virus, Canine parvovirus, Canine respiratory coronavirus, Classical swine fever virus, Eastern Equine encephalitis virus (EEE), Equine infectious anemia virus, Equine influenza virus, West nile virus, Feline Calictvirus, Feline enteric coronavirus, Feline immunodeficiency virus, Feline infectious peritonitis virus, Feline herpes Virus, Feline influenza virus, Feline leukemia virus (FeLV), Feline viral rhinotracheitis virus, Lentivirus, Marek's disease virus, Newcastle Disease virus, Ovine herpesviruses, Ovine parainfluenza 3, Ovine progressive pneumonia virus, Ovine pulmonary adenocarcinoma virus, Pantropic CCV, African swine fever virus, Classical swine fever virus, Foot and Mouth disease virus, Porcine coronaviruses, Porcine circovirus (PCV) Type I, PCV Type II, Porcine epidemic diarrhea virus, Porcine hemaggiutinating encephalomyletitis virus, Porcine herpesviruses, Porcine parvovirus, Porcine reproductive and respiratory syndrome (PRRS) Virus, Pseudorabies virus, Rabies, Rotavirus, Rhinoviruses, Rinderpest virus, Swine influenza virus, Transmissible gastroenteritis virus, Turkey coronavirus, Venezuelan equine encephalitis virus, Vesicular stomatitis virus, West Nile virus, Western equine encephalitis virus, *Adenoviridae* (most adenoviruses); *Arena viridae* (hemorrhagic fever viruses); Asfavirus (African swine fever virus as its only member) Astroviruses; *Bungaviridae* (e.g., *Hantaan* viruses, bunga viruses, phleboviruses and Nairo viruses); *Calciviridae* (e.g., Rabbit haemorrhagic virus and other viral strains that cause gastroenteritis); *Coronoviridae* (e.g., coronaviruses); Porcine Diarrheal virus, Porcine gastroenteritis virus, Middle East respiratory syndrome coronavirus (MERS-CoV), Severe acute respiratory syndrome coronavirus 1 (SARS-CoV-1), Severe acute respiratory syndrome coronavirus 2 (SARS-CoV-2), *Filoviridae* (e.g., ebola viruses); *Flaviridae* (e.g., Classical swine fever virus, hepatitis C virus, dengue viruses, encephalitis viruses, yellow fever viruses); *Hepadnaviridae* (Hepatitis B virus); *Herpesviridae* (Porcine Pseudorabies virus (Aujeszky), herpes simplex virus (HSV) 1 and 2, varicella zoster virus, cytomegalovirus (CIvIV), herpes virus); *Iridovtridae*; Norwalk and related viruses; *Orthomyxoviridae* (e.g., influenza viruses); *Papovaviridae* (papilloma viruses, polyoma viruses); *Paramyxoviridae* (e.g., parainfluenza viruses, mumps virus, measles virus, respiratory syncytial virus); *Parvoviridae* (parvoviruses); *Picomaviridae* (e.g., Foot and Mouth disease virus, polio viruses, hepatitis A virus; enteroviruses, human Coxsackie viruses, rhinoviruses, echovtruses); *Poxviridae* (variola viruses, vaccinia viruses, monkeypox virus and other pox viruses); *Reoviridae* (e.g., reoviruses, orbiviruses and rotaviruses); *Retroviridae* (e.g. human immunodeficiency viruses, such as HIV-1or HIV-2 (also referred to as HTLV-III, LAV or HTLV-III/LAV, or HIV-III; and other isolates, such as HI -LP); *Rhabdoviridae* (e.g., vesicular stomatitis viruses, rabies viruses); *Togaviridae* (e.g., equine encephalitis viruses, rubella viruses); and Unclassified entities such as the etiological agents of Spongiform encephalopathies, the agent of delta hepatitis (thought to be a defective satellite of hepatitis B virus).

In another particular embodiment, the infection to be treated or to be prevented with the strain of *Rothia nasimurium* of the invention, or immunomodulatory fraction thereof, is a parasitic infection. More preferably, the parasitic infection is caused by a parasite from the genus selected from the group consisting of: *Anaplasma, Ancylostoma* (hookworms), *Ascaris, Babesia, Coccidia, Cryptosporidium parvum, Dirofilaria* (heartworms), *Eimeria* species, *Fasciola hepatica* (liver fluke), *Giardia, Hammondia, Isopsora, Leishmania* species, *Neospora caninum, Plasmodium species, Sarcocystis, Schistosoma, Strongy ides, Taenia, Toxoplasma gondii, Trichineila species, Trichomonas species, Trypanosoma* species, and external parasites such as ticks, for example *Ixodes, Rhipicephalus, Dermacentor, Ambiyomma, Boophilus, Hyaiomma,* and *Haemaphysaiis* species.

The *Rothia nasimurium* or immunomodulatory fraction may be a vector.

In a particular embodiment, the genomic sequence of the strain of *Rothia nasimurium* for use in the treatment and/or prevention method of the instant invention has a sequence similarity of at least 85%, of at least 90%, of at least 95%, of at least 96%, of at least 97%, of at least 98%, of at least 99% with the genomic sequence of the strain of *Rothia nasimurium* with CECT accession number 30828. In a particular embodiment of the invention, the strain of *Rothia nasimurium* used in the treatment of an infection has CECT accession number 30828.

In the context of the invention, the cell of the microorganism may be alive or inactivated. That is, the cells of *Rothia nasimurium* may be alive, or inactivated. The fact that *Rothia nasimurium* is a commensal bacteria of the normal microbiota of the animals minimizes the risk of using alive cells here, as demonstrated with the lack of any clinical signs after inoculation *in vivo.* The term "inactivated" refers to a dead or inactivated cell of a microorganism which is no longer capable of growing to form a single colony on a plate having medium specific for said microorganism. This term also includes lysates, fractions and/or extracts of the microorganism. The term "inactivation", as used herein, refers to the process of transforming a cell from viable to non-viable. The term "viable", "viability" and the like, refers to the ability of a cell to maintain itself and survive until it is able to divide. Thus, a cell that is viable indicates that the cell is able to survive and divide; conversely, a cell that is non-viable indicates that the cell is not able to survive and divide. Accordingly, non-viable cells include dead cells. Viability can be determined by a number of assays that are conventional in the art. These include, but are not limited to: Tetrazolium Reduction Assay; Resazurin Reduction Assays; Protease Viability Marker Assay; ATP Assay (e.g., the "firefly luciferase" method); Assay of sodium-potassium ratio; Cytolysis or membrane leakage (e.g., lactate dehydrogenase assay; propidium iodide assay, trypan blue assay, and 7-Aminoactinomycin D assay); Mitochondrial activity or caspase (e.g., Resazurin and Formazan; apoptosis assay); Functional Assays of cell function (e.g., motility, deformability, osmotic fragility, hemolysis, ATP level, hemoglobin content, etc.); Genomic and proteomic assays; Flow Cytometry-based assays.

The live or inactivated strain of *Rothia nasimurium* of the invention, or immunomodulatory fraction thereof, has particular utility in treating disease and/or preventing development of a disease in a mammal, such as: wild animals (e.g., apes, monkey, rat, mouse, rabbit and boar), farm animals (e.g., cattle, horses, goats, sheep and pigs), household pets including cats and dogs, and humans. Therefore, the term "subject" in the context of the present invention preferably refer to any mammal including, without limitation, apes, monkeys, rats, mice, rabbits, boars, cattle, horses, goats, sheep, pigs, cats, dogs and humans. In a more preferred embodiment, the subject is a boar (i.e., *Sus scrofa*) and/or a domestic pig (i.e., *Sus scrofa domestica*). In preferred embodiments, the subject is a mammal in any stage of development, such as a pre-weaning infant, a post-weaning infant, or an adult. In a preferred embodiment, the subject is an adult mammal.

The live or inactivated strain of *Rothia nasimurium* of the invention, or immunomodulatory fraction thereof, for use in the prevention and/or treatment of an infection can be provided by any route of administration, including enteral (wherein the medicament is delivered through the gastrointestinal tract), nasal (wherein the medicament is delivered through the respiratory mucosa), topical (onto skin or mucous membranes) or parenteral (wherein the medicament is delivered by routes other than the gastrointestinal tract such as intradermal, subcutaneous, intravenous, intramuscular, and intraperitoneal). Examples include, without limitation, oral, sublingual, intravenous, intranasal, intraperitoneal, mucosal, intrapulmonary, enteral, parenteral, topical, rectal route or combinations thereof. A person skilled in the art should be capable of selecting an appropriate route of administration for the live or inactivated strain of *Rothia nasimurium* of the invention, or immunomodulatory fraction thereof. A review of the different forms for the administration of active ingredients can be found in Remington's The Science and Practice of Pharmacy (Adeboye Adejare, Editor-in-Chief), 23rd edition, Elsevier (2020). Thus, in a preferred embodiment the live or inactivated strain of *Rothia nasimurium* of the invention, or immunomodulatory fraction thereof, for use in the prevention and/or treatment of any infectious or non-infectious disease can be administered by oral, sublingual, intravenous, intranasal, intraperitoneal, intradermal, subcutaneous, mucosal, intrapulmonary, enteral, parenteral, topical, rectal route or combinations thereof. In a particular embodiment of the invention, the live or inactivated strain of *Rothia nasimurium* of the invention, or immunomodulatory fraction thereof, for use in the prevention and/or treatment of a disease is administered by intranasal inoculation.

In a particular embodiment of the invention, the live or inactivated strain or immunomodulatory fraction thereof for use in the prevention and/or treatment of a disease further comprises an adjuvant. The term "adjuvant", as used herein, refers to a substance which, when added to an immunogenic agent, non-specifically enhances or potentiates an immune response to the agent in a recipient host upon exposure to the mixture. Suitable adjuvants are within the reach of the person skilled in the instant technical field.

In an embodiment, the live or inactivated strain or immunomodulatory fraction thereof for use in the prevention and/or treatment of an infection is not provided together with any antigen derived from an infectious agent. Preferably, the live or inactivated strain or immunomodulatory fraction thereof is not accompanied with an antigen derived from the infectious or non-infectious agent that causes the disease to be treated and/or prevented. In another embodiment live or inactivated *Rothia nasimurium* or immunomodulatory fraction is the only component that is provided to the subject in need thereof.

In particular embodiments of the invention, the live or inactivated strain or immunomodulatory fraction thereof can be administered as part of a product selected from the group consisting of a pharmaceutical product, a veterinary product, a feed product or feed additive, or a nutritional product, as well as a prebiotic, probiotic or postbiotic product. In a related aspect, the invention also refers to a pharmaceutical product, a veterinary product, a feed product or feed additive, or a nutritional product, as well as a prebiotic, probiotic or postbiotic product, wherein the product comprises a live or inactivated strain of *Rothia nasimurium,* or immunomodulatory fraction thereof, preferably wherein the strain of *Rothia nasimurium* is the strain of *Rothia nasimurium* as defined in the instant invention, with CECT accession number 30828. The pharmaceutical product, veterinary product, feed product, or nutritional product according to the invention may be provided in any suitable format, known to the skilled person, in particular embodiments, the pharmaceutical product, veterinary product, feed product, or nutritional product according to the invention may be provided in multiples forms, such as a lyophilized, freeze-dried or dried form, which can be obtained by any conventional method known in the art.

As used in the present invention, the expression "pharmaceutical product" refers to a formulation which has been designed to comprise a predetermined dose of one or several therapeutically useful agents, such that a therapeutic effect may be achieved by administration of said pharmaceutical product to a subject in need thereof. A pharmaceutical product in the context of the instant invention comprises a pharmaceutically effective amount of the live or inactivated strain of *Rothia nasimurium,* or immunomodulatory fraction thereof, wherein the effective amount is capable of providing a therapeutic effect, and which can be determined by the person skilled in the art by commonly used means. The amount of the live or inactivated strain of *Rothia nasimurium* for use according to the invention or a fraction thereof will vary depending upon the subject and the particular mode of administration. A person skilled in the art of the invention will readily understand that effective amounts may also be determined with guidance from Goodman and Goldman's The Pharmacological Basis of Therapeutics, Fourteenth Edition (2022).

The pharmaceutical product may be for human or animal usage in human and veterinary medicine and will typically comprise any one or more of a pharmaceutically acceptable carrier. The term "carrier" refers to a diluent or excipient with which the active ingredient is administered. Acceptable carriers or diluents for therapeutic use are well known in the pharmaceutical art. The choice of pharmaceutical carrier can be selected with regard to the intended route of administration and standard pharmaceutical practice. There may be different formulation requirements depending on the different delivery systems.

In particular embodiments, the composition is administered as part of a nutritional or nutraceutical product comprising the live or inactivated strain of *Rothia nasimurium,* or immunomodulatory fraction thereof, for use according to the invention. The term "nutritional or nutraceutical product" in connection with the present invention relates to a food product that beneficially affects one or more functions of the body, so as to provide better health. Accordingly, such a nutritional or nutraceutical product may be intended for the prevention and/or treatment of a disease or a disease-causing factor. Therefore, the term "nutritional or nutraceutical product" in the context of the present invention can be used as a synonym for functional food or foods for particular nutritional purposes, or medical food. A nutritional or nutraceutical product may be similar in composition to that of conventional food products and consumed as part of a normal diet. Preferably, the feed or nutritional product comprises at least between 0.1 % and 99.9%, between 1 % and 99%, between 10% and 90%, between 20% and 80%, between 30% and 70%, between 40% and 60% of the live or inactivated strain of *Rothia nasimurium* for use according to the invention, or the immunomodulatory fraction thereof. Non-limiting examples of suitable foodstuffs which can be used in the present invention are cereals, fermented cereal-based products, other cereal based powders, clinical nutrition formula, bread, cakes or candies, animal feed formulations, semi- or synthetic diet formulations, infant formulae, clinical nutrition formulae, flours, bread, cakes, candies or chewing-gums. In the context of the instant invention, the term "nutraceutical", which is derived from the words "nutrition" and "pharmaceutical", refers to products made from food components, but which can be found in pill form, a powder and/or any other dosage forms not usually associated with food and having beneficial properties for the treatment and/or prevention of diseases.

In a particular embodiment the live or inactivated strain of *Rothia nasimurium,* or immunomodulatory fraction thereof, is administered as part of an animal feed. In the context of the invention, the term "animal feed" refers to nourishment supplied to non-human animals, and includes materials of suitable, nutritious nature to be consumed by an animal. A typical animal feed for an animal may further comprise concentrates as well as vitamins, minerals, amino acid and/or other feed ingredients. Animal feed may be provided as pellets or in any appropriate form.

In a further aspect, the invention refers to the use of a live or inactivated strain of *Rothia nasimurium,* or immunomodulatory fraction thereof, as an adjuvant, particularly as a vaccine adjuvant. That is, in an embodiment, the strain, or immunomodulatory fraction thereof, is provided as a component forming part of a vaccine or a vaccine composition. In a preferred embodiment, the strain of *Rothia nasimurium* has CECT accession number 30828.

In another aspect, the invention refers to a composition comprising:
(a) a live attenuated African swine fever virus (ASFV); and
(b) a live or inactivated strain of *Rothia nasimurium,* or immunomodulatory fraction thereof.

In particular embodiments of the invention, the live attenuated African swine fever virus (ASFV) of the composition of the invention is a recombinant live African swine fever virus (ASFV) lacking the CD2v antigen (BA71ΔCD2), or a recombinant live African swine fever virus (ASFV) lacking the CD2v antigen and the UK antigen (BA71ΔCD2ΔUK). In particular embodiments, the strain of *Rothia nasimurium* has CECT accession number 30828.

In a final aspect, the invention refers to a composition according to the invention for use in the prevention and/or treatment of African Swine Fever (ASF). African Swine Fever (ASF) presents clinical symptoms that are very similar to classical swine fever, wherein laboratory tests may be necessary to distinguish between the two diseases. The tests may involve ELISA, real time PCR or isolation of the virus from blood, lymph nodes, spleen, or serum from an infected pig. Highly virulent strains of ASFV may cause an acute form of the disease, wherein pigs may develop a high fever, but show no other noticeable symptoms during the first few days. As the acute form of the disease progresses, the pigs gradually lose their appetite and may become depressed. Animal extremities may turn purple and haemorrhages on ears and abdomen may become visually noticeable in white or lighter skinned pigs. Infected pigs may also show signs of shivering, coughing and abnormal breathing. The disease may progress to a comatose state and result in death of the infected pig. In pregnant sows, spontaneous abortions may occur. ASFV may cause a milder form of the infection, wherein affected pigs lose weight, become thin, and develop signs of pneumonia, skin ulcers, and swollen joints.

All the terms and embodiments described in the instant document are equally applicable to all aspects of the invention. It should be noted that, as used in the specification and in the appended claims, the singular forms "a", "an", and "the" include their plural referents unless the context clearly indicates otherwise. Similarly, the term "comprises" or "comprising" as used herein also describes "consists of' or "consisting of' in accordance with generally accepted patent practice.

### EXAMPLES

The instant invention is hereby described by way of the following examples, which are to be construed as merely illustrative and not limitative of the scope of the invention.

### Example 1: Rothia nasimurium is present in warthog microbiota

Recent work performed at the "Centre de Recerca en Sanitat Animal" (CReSA), has demonstrated that ASF susceptibility depends not only on genetics, but also on environmental factors. Thus, genetically identical pigs, grown in conventional farms or in specific pathogen free conditions (SPF), showed different susceptibility to infection with ASFV. Taking into account that gut microbiota, a key component regulating the immune system (and many other physiological aspects), is also affected by both genetic and environmental factors, the inventors decided to study the effects of faecal transplantation from warthogs obtained from the ZOO of Barcelona (*Phacochoerus africanus*), a pig species that is highly resistant to ASF, to highly susceptible domestic pigs (Sus *scrofa*). It was observed that faecal transplants from warthog to pig, but not from pig to pig, yielded domestic pigs more resistant to infection with attenuated strains of ASFV. The protection afforded against ASFV correlated with a significant increase of total Immunoglobulin A (IgA) in sera, indicating a potentiation of their mucosal immunity. More recent work allowed isolating more than 250 individual bacteria from warthog faeces microbiota, many of them with beneficial properties *in vitro* (Zhang et al., "Fecal microbiota transplantation from warthog to pig confirms the influence of the gut microbiota on African swine fever susceptibility". Sci Rep. 2020 Oct 19;10(1):17605). In these regards, several bacterial components from the microbiota (from different genera) showed microbicide properties against pathogenic bacteria, including *Salmonella, E. coli, Clostridium perfringens,* and *Streptococcus suis,* among others. Other microbiota components improved the viability of intestinal organoids grown *in vitro,* while others were able to stimulate gut associated lymphoid tissue (GALT) cells to secrete different immunoregulatory molecules. Occasionally, one single bacterium showed more than one of these capabilities, requiring further characterization. Intra-gastric inoculation of 15 bacterial isolates selected according to their *in vitro* capabilities, mimicked the results obtained with the faecal transplant, including their ability to improve the mucosal immune responses.

One of the most interesting isolates was further characterized. The total DNA was extracted from the isolated bacteria (D24) and submitted for whole genome sequencing with Illumina MiSeq technology. Moreover, the DNA was used to perform a PCR of the 16S gene to identify the bacterial isolate (primers 8F and 1492R). The sequence was analyzed using BLAST and the bacterium was classified as *Rothia nasimurium.* The draft genome was aligned against several representative genomes from this genus and a phylogenetic tree was built with RAxML to confirm the classification (Figure 1). The *Rothia nasimurium* D24 genome was annotated using RAST tool kit. The taxonomy of this genome is *Bacteria* > *Terrabacteria group* > *Actinobacteria* > *Actinobacteria* > *Micrococcales* > *Micrococcaceae* > *Rothia* > *Rothia nasimurium.*

In view of the genome data, no antimicrobial resistance was detected, using ResFinder. A summary of the AMR genes annotated in this genome and corresponding class and WGS-predicted phenotype is provided in Table 1 below. The phenotype 'No resistance' should be interpreted with caution, as it only means that nothing in the used database indicate resistance, but resistance could exist from 'unknown' or not yet implemented sources.

**Table 1**

| **# Antimicrobial** | **Class** | **WGS-predicted phenotype** |
|---|---|---|
| tiamulin | pleuromutilin | No resistance |
| tetracycline | tetracycline | No resistance |
| minocycline | tetracycline | No resistance |
| doxycycline | tetracycline | No resistance |
| tigecycline | tetracycline | No resistance |
| fosfomycin | fosfomycin | No resistance |
| rifampicin | rifamycin | No resistance |
| quinupristin+dalfopristin | streptogramin a | No resistance |
| pristinamycin iia | streptogramin a | No resistance |
| dalfopristin | streptogramin a | No resistance |
| virginiamycin m | streptogramin a | No resistance |
| trimethoprim | folate pathway antagonist | No resistance |
| sulfamethoxazole | folate pathway antagonist | No resistance |
| ceftiofur | under_development | No resistance |
| quinupristin | streptogramin b | No resistance |
| virginiamycin s | streptogramin b | No resistance |
| pristinamycin ia | streptogramin b | No resistance |
| benzylkonium chloride | quaternary ammonium compound | No resistance |
| chlorhexidine | quaternary ammonium compound | No resistance |
| ethidium bromide | quaternary ammonium compound | No resistance |
| cetylpyridinium chloride | quaternary ammonium compound | No resistance |
| formaldehyde | aldehyde | No resistance |
| metronidazole | nitroimidazole | No resistance |
| fusidic acid | steroid antibacterial | No resistance |
| hydrogen peroxide | peroxide | No resistance |
| lincomycin | lincosamide | No resistance |
| clindamycin | lincosamide | No resistance |
| oleandomycin | macrolide | No resistance |
| tylosin | macrolide | No resistance |
| azithromycin | macrolide | No resistance |
| telithromycin | macrolide | No resistance |
| carbomycin | macrolide | No resistance |
| erythromycin | macrolide | No resistance |
| spiramycin | macrolide | No resistance |
| mupirocin | pseudomonic acid | No resistance |
| spectinomycin | aminocyclitol | No resistance |
| ticarcillin | beta-lactam | No resistance |
| cefotaxime | beta-lactam | No resistance |
| ceftriaxone | beta-lactam | No resistance |
| ceftazidime+avibactam | beta-lactam | No resistance |
| amoxicillin+clavulanic acid | beta-lactam | No resistance |
| piperacillin+clavulanic acid | beta-lactam | No resistance |
| piperacillin+tazobactam | beta-lactam | No resistance |
| unknown beta-lactam | beta-lactam | No resistance |
| penicillin | beta-lactam | No resistance |
| piperacillin | beta-lactam | No resistance |
| ampicillin+clavulanic acid | beta-lactam | No resistance |
| meropenem | beta-lactam | No resistance |
| cefotaxime+clavulanic acid | beta-lactam | No resistance |
| cefepime | beta-lactam | No resistance |
| cefoxitin | beta-lactam | No resistance |
| imipenem | beta-lactam | No resistance |
| ceftazidime | beta-lactam | No resistance |
| cephalothin | beta-lactam | No resistance |
| ertapenem | beta-lactam | No resistance |
| ticarcillin+clavulanic acid | beta-lactam | No resistance |
| temocillin | beta-lactam | No resistance |
| cephalotin | beta-lactam | No resistance |
| aztreonam | beta-lactam | No resistance |
| cefixime | beta-lactam | No resistance |
| amoxicillin | beta-lactam | No resistance |
| ampicillin | beta-lactam | No resistance |
| linezolid | oxazolidinone | No resistance |
| nalidixic acid | quinolone | No resistance |
| fluoroquinolone | quinolone | No resistance |
| unknown quinolone | quinolone | No resistance |
| ciprofloxacin | quinolone | No resistance |
| florfenicol | amphenicol | No resistance |
| chloramphenicol | amphenicol | No resistance |
| colistin | polymyxin | No resistance |
| teicoplanin | glycopeptide | No resistance |
| vancomycin | glycopeptide | No resistance |
| astromicin | aminoglycoside | No resistance |
| butiromycin | aminoglycoside | No resistance |
| apramycin | aminoglycoside | No resistance |
| butirosin | aminoglycoside | No resistance |
| lividomycin | aminoglycoside | No resistance |
| paromomycin | aminoglycoside | No resistance |
| fortimicin | aminoglycoside | No resistance |
| neomycin | aminoglycoside | No resistance |
| kasugamycin | aminoglycoside | No resistance |
| sisomicin | aminoglycoside | No resistance |
| gentamicin | aminoglycoside | No resistance |
| ribostamycin | aminoglycoside | No resistance |
| streptomycin | aminoglycoside | No resistance |
| kanamycin | aminoglycoside | No resistance |
| unknown aminoglycoside | aminoglycoside | No resistance |
| dibekacin | aminoglycoside | No resistance |
| hygromycin | aminoglycoside | No resistance |
| bleomycin | aminoglycoside | No resistance |
| netilmicin | aminoglycoside | No resistance |
| arbekacin | aminoglycoside | No resistance |
| isepamicin | aminoglycoside | No resistance |
| tobramycin | aminoglycoside | No resistance |
| amikacin | aminoglycoside | No resistance |

### Example 2: In vitro stimulation with warthog Rothia nasimurium and/or IM fraction thereof promotes secretion of pro and anti-inflammatory cytokines

*In vitro* work with the *Rothia nasimurium* D24 isolate (hereinafter named as *Rothia*) confirmed its *in vitro* immunomodulatory abilities, being capable to stimulate the secretion of IFNγ by cells from different origins. A first set of experiments using a newborn pig trachea cell line (NPTr) confirmed that *Rothia's* immunomodulatory abilities were dose dependant (Figure 2A). The inventors next confirmed that this effect was reproducible using primary porcine cells, including peripheral blood mononuclear cells (PBMCs) and a mixed preparation of lymphocytes from Peyer's patches (PP) and mesenteric lymph nodes (MSLN) (Figures 2B and 2C, respectively).

Aiming to better characterize the immunomodulatory abilities of the warthog *Rothia,* the inventors concentrated their efforts to study these effects in primary porcine alveolar macrophages (PAMs), a relevant *in vitro* model to study innate immunity and main target for ASFV infection. The inventors observed the ability of alive warthog *Rothia* to stimulate PAMs to secrete IFNγ (*Rothia* in Figure 3A), and interestingly this effect was maintained once *Rothia* was heat inactivated. Importantly, this immunomodulatory capacity is also reproduced when using what the inventors have called *"Rothia's* wash" (RW), obtained after collecting in PBS or culture media (RPMI) the *Rothia* colonies grown on agar plates, then centrifuged to pellet the bacteria and finally filtered through a 0.2 µ filter (RW in Figure 3A). A simple fractionation of the RW using Amicon Ultra-15 Centrifugal Filter Unit columns (Millipore #UFC9100), allowed the inventors to confirm that the RW fraction of higher molecular weight (>100KDa in Figure 3A) maintains the IM capabilities, while RW fractions with molecular weights between 10-100KDa and <10KDa were not able to stimulate PAMs to secrete IFNγ (Figure 3A). Exactly these same results were observed when measuring tumour necrosis factor alfa (TNFα) in the same PAMs supernatants (Figure 3B), confirming the IM nature of warthog *Rothia* and the high molecular weight fraction (>100KDa) of RW.

A more profound experimental characterization demonstrated that both alive *Rothia* and the immunomodulatory fraction, are able to *in vitro* stimulate PAMs not only to secrete IFNγ and TNFα, but also other pro-inflammatory and anti-inflammatory cytokines, detectable by LUMINEX (Labclinics S.A.), and in many cases, in a statistically significant manner (Figure 4).

### Example 3: The Th1-like immune responses induced after intranasal inoculation of BA71ΔCD2ΔUK is improved by using warthog Rothia nasimurium as adjuvant

With this data at hand, the potential use of warthog *Rothia* as a vaccine adjuvant was explored. To address this issue, the inventors decided to use a recombinant LAV (BA71ΔCD2ΔUK) obtained in their laboratory by the doble depletion of the CD2v and UK genes from the parental ASFV strain BA71 as an *in vivo* model. This live attenuated vaccine (LAV) was initially discarded for field use due to the suboptimal immune responses induced in pigs upon intramuscular immunization. However, the inventors aimed to test the ability of warthog *Rothia* to enhance the immune responses induced by BA71ΔCD2ΔUK. The first *in vivo* experiment consisted of comparing the immune responses induced after intranasal inoculation of 10⁵ plaque forming units (PFU) of BA71ΔCD2ΔUK in the absence or presence of different potential adjuvants. Briefly, groups of 6 pigs were vaccinated with either BA71ΔCD2ΔUK alone (group 1), or with same amount of BA71ΔCD2ΔUK combined with the following adjuvants:
i. 10⁷ colony forming units (CFU) of alive warthog *Rothia* (group 2),
ii. the immunomodulatory fraction of RW, i.e., the high molecular weight fraction (>100KDa) of RW (group 3), or
iii. CAF01 (Group 4), a Th1-adjuvant successfully used in clinical trials in humans and experimentally in pigs.

A fifth group of pigs inoculated with PBS was included as control group. Sera samples were taken at 0, 7 and 14 days post-infection to compare the kinetics of ASFV-specific antibody induction by ELISA, and PBMCs were obtained at the latter point (coinciding with the end of the study) to analyze the ASFV-specific T-cell responses by using an IFNγ ELISPOT. Despite no significant differences were observed regarding the ASFV-specific IgG induced (Figure 5A), the presence of both alive warthog *Rothia* and immunomodulatory fraction clearly improved the Th1 ASFV-specific T-cell responses induced by BA71ΔCD2ΔUK (Figure 5B).

Interestingly, the presence of CAF01 adjuvant abolished the humoral responses induced by BA71ΔCD2Δ and the specific T-cell responses induced did not show any dramatic improvement (Figure 5), in line with results previously obtained in the laboratory with other commercial adjuvants. Adjuvants and immune-stimulators are crucial to fighting almost any disease and not knowing barrier species. There are many immune-stimulators and adjuvants companies both veterinary and human pharmaceuticals that are avid of solutions that will result in a severe reduction of antibiotic usage.

It is worth to mention, that the presence of *Rothia* did not produce any toxic effect *in vitro,* nor *in vivo,* even after several administrations either intragastrically, intranasally or intramuscularly, and that the general mucosal immunity (total IgA detected) seems to improve, indicating a generalized immunomodulatory ability. These results strongly suggest the potential use of *Rothia nasimurium* or of the IM fraction thereof in vaccines but also as immunostimulants (not associated to a specific vaccine as an adjuvant), or the use of *Rothia nasimurium* as a probiotic or as a feed additive.

## Claims

1. A strain of *Rothia nasimurium* or a fraction thereof, **characterised in that** the strain of *Rothia nasimurium* has CECT accession number 30828, and wherein the strain or the fraction thereof have immunomodulatory properties.

2. The strain of *Rothia nasimurium* or a fraction thereof according to claim 1, wherein the strain is a live strain or an inactivated strain.

3. The strain of *Rothia nasimurium* or a fraction thereof according to any one of claims 1 or 2, wherein has a sequence similarity with the genomic sequence of the strain of *Rothia nasimurium* with CECT accession number 30828 selected from the group of percentages consisting of: at least 70%, at least 75%, at least 80%, at least 85%, at least 90%, at least 95%, at least 96%, at least 97%, at least 98%, and at least 99%.

4. A strain of *Rothia nasimurium* or a fraction thereof according to any one of claims 1 to 3, for use in medicine.

5. The strain or fraction thereof for the use according to claim 4, in the prevention and/or treatment of an infection in a subject, or in the prevention and/or treatment of non-infectious diseases.

6. The strain or fraction thereof for the use according to any one of claims 4 or 5, wherein the subject is a mammal.

7. The strain or fraction thereof for the use according to any one of claims 4 to 6, wherein it is administered by a route of administration selected from the group consisting of: intranasal inoculation, oral, sublingual, intravenous, intraperitoneal, mucosal, intrapulmonary, enteral, parenteral, topical, rectal route and any combination thereof.

8. The strain or fraction thereof for the use according to any one of claims 4 to 7, wherein it further comprises an adjuvant.

9. The strain or fraction thereof for the use according to any one of claims 4 to 8, wherein the strain or immunomodulatory fraction thereof is administered as part of a pharmaceutical product, a veterinary product, a feed product, or a nutritional product.

10. A pharmaceutical product, a veterinary product, a feed product, a feed additive, a nutritional product or a probiotic product, wherein the product comprises a strain of *Rothia nasimurium,* or fraction thereof according to any one of claims 1 to 3.

11. Use of a strain of *Rothia nasimurium,* or fraction thereof according to any one of claims 1 to 3, as an adjuvant.

12. A composition comprising:
(a) a live attenuated African swine fever virus (ASFV); and
(b) a live or inactivated strain of *Rothia nasimurium,* or a fraction thereof.

13. The composition according to claim 12, wherein the live attenuated African swine fever virus is a recombinant live African swine fever virus lacking the CD2v antigen (BA71ΔCD2), or a recombinant live African swine fever virus lacking the CD2v antigen and the UK antigen (BA71ΔCD2ΔUK).

14. The composition according to any one of claims 12 or 13, wherein the strain of *Rothia nasimurium* has CECT accession number 30828.

15. A composition according to any one of claims 12 to 14, for use in the prevention and/or treatment of African Swine Fever.

## Amended claims

### Amended claims in accordance with Rule 137(2) EPC.

1. A strain of *Rothia nasimurium* or an immunomodulatory fraction thereof, **characterised in that** the strain of *Rothia nasimurium* has CECT accession number 30828.

2. The strain of *Rothia nasimurium* or the immunomodulatory fraction thereof according to claim 1, wherein the strain is a live strain or an inactivated strain.

3. The strain of *Rothia nasimurium* or the immunomodulatory fraction thereof according to any one of claims 1 or 2, wherein has a sequence similarity with the genomic sequence of the strain of *Rothia nasimurium* with CECT accession number 30828 selected from the group of percentages consisting of: at least 70%, at least 75%, at least 80%, at least 85%, at least 90%, at least 95%, at least 96%, at least 97%, at least 98%, and at least 99%.

4. A strain of *Rothia nasimurium* or an immunomodulatory fraction thereof according to any one of claims 1 to 3, for use in medicine.

5. The strain or immunomodulatory fraction thereof for the use according to claim 4, in the prevention and/or treatment of an infection in a subject, or in the prevention and/or treatment of non-infectious diseases.

6. The strain or the immunomodulatory fraction thereof for the use according to any one of claims 4 or 5, wherein the subject is a mammal.

7. The strain or the immunomodulatory fraction thereof for the use according to any one of claims 4 to 6, wherein it is administered by a route of administration selected from the group consisting of: intranasal inoculation, oral, sublingual, intravenous, intraperitoneal, mucosal, intrapulmonary, enteral, parenteral, topical, rectal route and any combination thereof.

8. The strain or the immunomodulatory fraction thereof for the use according to any one of claims 4 to 7, wherein it further comprises an adjuvant.

9. The strain or the immunomodulatory fraction thereof for the use according to any one of claims 4 to 8, wherein the strain or the immunomodulatory fraction thereof is administered as part of a pharmaceutical product, a veterinary product, a feed product, or a nutritional product.

10. A pharmaceutical product, a veterinary product, a feed product, a feed additive, a nutritional product or a probiotic product, wherein the product comprises a strain of *Rothia nasimurium,* or an immunomodulatory fraction thereof according to any one of claims 1 to 3.

11. Use of a strain of *Rothia nasimurium,* or an immunomodulatory fraction thereof according to any one of claims 1 to 3, as a vaccine adjuvant.

12. A composition comprising:
(a) a live attenuated African swine fever virus (ASFV); and
(b) a live or inactivated strain of *Rothia nasimurium,* or a fraction thereof.

13. The composition according to claim 12, wherein the live attenuated African swine fever virus is a recombinant live African swine fever virus lacking the CD2v antigen (BA71ΔCD2), or a recombinant live African swine fever virus lacking the CD2v antigen and the UK antigen (BA71ΔCD2ΔUK).

14. The composition according to any one of claims 12 or 13, wherein the strain of *Rothia nasimurium* has CECT accession number 30828.

15. A composition according to any one of claims 12 to 14, for use in the prevention and/or treatment of African Swine Fever.
